# EUROPÄISCHE PATENTSCHRIFT

(11) **EP 3 763 441 B1**
(45) Veröffentlichungstag und Bekanntmachung des Hinweises auf die Patenterteilung: **07.02.2024**
(21) Anmeldenummer: 20179167.0
(22) Anmeldetag: 10.06.2020
(51) Int. Cl.: B01L 3/00, B01L 9/06, A61B 50/30, A61B 50/31, A61B 50/36, B65D 30/24, A61B 50/00

(54) **TRANSPORTSYSTEM FÜR DIAGNOSTISCHE PROBEN**
TRANSPORT SYSTEM FOR DIAGNOSTIC SAMPLES
SYSTÈME DE TRANSPORT POUR ÉCHANTILLONS DIAGNOSTIQUES

(30) Priorität: 10.07.2019 DE 102019210174
(43) Veröffentlichungstag der Anmeldung: 13.01.2021
(73) Patentinhaber: T&O LabSystems GmbH & Co. KG, 24568 Kaltenkirchen (DE)
(72) Erfinder: Lorenzen, Dave, 24568 Kaltenkirchen (DE); Lorenzen, Tom, 24568 Kaltenkirchen (DE)
(74) Vertreter: Patentanwälte Vollmann Hemmer Lindfeld Partnerschaft mbB

(56) Entgegenhaltungen:
- DE-A1-102016 108 380
- DE-U1- 20 010 412
- US-A1- 2002 088 723
- US-A1- 2012 265 336

## Beschreibung

Die vorliegende Offenbarung betrifft ein Transportsystem für diagnostische Proben und ein Verfahren zum Sammeln und Transportieren von diagnostischen Proben. Dies betrifft insbesondere eine Mehrzahl von Blut-, Urin- oder Stuhlproben, die gesammelt zur medizinischen Untersuchung in ein Labor transportiert werden müssen.

Diagnostische Proben dieser Art werden hauptsächlich in Arztpraxen und Krankenhäusern in Form von Blutprobenröhrchen einer Mehrzahl von Patienten entnommen. Um recht zeitnah nach der Entnahme die Blutprobenröhrchen zur Untersuchung in ein Analyselabor zu schicken, werden die Blutprobenröhrchen in der Regel ein- oder mehrmals täglich durch Kurierdienste abgeholt und zum Analyselabor gebracht.

Die DE 10 2016 108 380 A1 beschreibt ein Transportsystem für diesen Zweck. Dabei werden die Proben als Schüttgut ungeordnet in einem Transportbehälter beim Arzt gesammelt. Bei Abholung des Transportbehälters durch den Kurier wird der Transportbehälter mit einer bodenseitigen Öffnung in eine Vertiefung eines Sammelbehälters gesteckt, wodurch sich der Transportbehälter bodenseitig öffnet, sodass die Proben aus dem Transportbehälter in den Sammelbehälter fallen. Der Sammelbehälter mit den Proben wird dann zum Analyselabor transportiert, während der geleerte Transportbehälter wieder zurück zum Arzt gebracht wird.

Das aus der DE 10 2016 108 380 A1 bekannte Transportsystem hat allerdings gewisse Nachteile. Zum einen kann der Kurier nicht schnell und einfach die Anzahl der als Schüttgut ungeordnet im Transportbehälter befindlichen Proben zählen, um deren Erhalt zu quittieren. Zum anderen kann es passieren, dass sich die ungeordnet im Transportbehälter befindlichen Proben gegenseitig blockieren und unbemerkt nicht alle Proben in den Sammelbehälter durch die bodenseitige Öffnung fallen. Schließlich ist der Transportbehälter nicht sicher verschlossen gegen eine unautorisierte Öffnung des Deckels oder der bodenseitigen Öffnung.

Daraus ergibt sich die Aufgabe, ein sichereres und einfacher handhabbares Transportsystem zur Verfügung zu stellen.

Gemäß einem ersten Aspekt der vorliegenden Offenbarung wird zur Lösung dieses Problems ein Transportsystem für diagnostische Proben bereitgestellt mit einem Sammelbehälter und mindestens einem mobilen Transportbehälter. Der Transportbehälter weist dabei eine verschließbare Oberseite auf, wobei der Sammelbehälter eine oberseitige Öffnung aufweist, die mindestens so groß ist wie die Oberseite des Transportbehälters, wobei der Transportbehälter zum Ausleeren von diagnostischen Proben in den Sammelbehälter umgedreht mit verschlossener Oberseite nach unten auf die oberseitige Öffnung des Sammelbehälters in eine Ausleerposition platzierbar ist, wobei die verschlossene Oberseite des Transportbehälters nur in der Ausleerposition ohne ein Hilfsmittel öffenbar ist und außerhalb der Ausleerposition nur mit dem Hilfsmittel öffenbar ist.

Solange die verschließbare Oberseite offen ist, können die Proben beim Arzt oder im Krankenhaus in geordneter Ausrichtung, vorzugsweise im Wesentlichen parallel zueinander in einer im Wesentlichen vertikalen Ausrichtung im Transportbehälter angeordnet werden. Dadurch sind die Proben für den Kurier leicht zählbar, sodass der Kurier den Erhalt einer bestimmten Anzahl an Proben einfach und schnell quittieren kann. Wenn ein Deckelelement zum Verschließen der Oberseite vorzugsweise durchsichtig ausgestaltet ist, kann der Transportbehälter bereits vor Eintreffen des Kuriers verschlossen werden. Ansonsten kann der Kurier die Proben bei geöffneter Oberseite zählen und erst dann die Oberseite für den Transport verschließen. Außerdem ist die geordnete Ausrichtung der Proben vorteilhaft, da beispielsweise Blutproben nach der Blutentnahme mindestens 10 bis 15 Minuten aufrecht gelagert werden sollen, um den Gerinnungsprozess nicht zu fördern. Die Proben können also direkt in den Transportbehälter eingelagert und müssen nicht aufrecht zwischengelagert werden. Zum Verschließen ist vorzugsweise kein Hilfsmittel notwendig. Der Transportbehälter schnappt beim Verschließen der Oberseite vorzugsweise selbsttätig zu. Zum zerstörungsfreien Öffnen außerhalb der Ausleerposition ist allerdings ein Hilfsmittel notwendig. Damit ist der Transportbehälter gegen unautorisierte Öffnung gesichert.

Wird allerdings der Transportbehälter in die Ausleerposition gebracht, ist das Hilfsmittel hingegen zum Öffnen der Oberseite nicht notwendig. Das Hilfsmittel wird daher beim gewöhnlichen Gebrauch überhaupt nicht benötigt, sondern nur in Ausnahmefällen, wenn der Transportbehälter außerhalb der Ausleerposition geöffnet werden muss. Die Ausleerposition ist eine eindeutig definierte Position an der oberseitigen Öffnung des Sammelbehälters, auf welche der Transportbehälter mit eindeutig definierter Ausrichtung umgedreht mit der Oberseite nach unten gesetzt wird. Der Sammelbehälter kann sich beispielsweise im Transportwagen des Kuriers befinden. Der Kurier bringt einen oder mehrere Transportbehälter vom Abholort zum Sammelbehälter im Transportwagen und entleert sie darin. Der oder die Transportbehälter können dann entleert für die weitere Verwendung beim Arzt zum Abholort zurückgebracht werden. Alternativ oder zusätzlich kann der Kurier gleich leere Transportbehälter mit auf den Weg zum Abholort der gefüllten Transportbehälter nehmen und am Abholort gefüllte Transportbehälter durch leere Transportbehälter eintauschen und die gefüllten Transportbehälter zur Entleerung zum Sammelbehälter mitnehmen. Die geleerten Transportbehälter müssen dann nicht mehr zurückgebracht werden und können beim nächsten Abholort verwendet werden. Der Sammelbehälter mit den als Schüttgut ungeordnet gesammelten Proben wird dann zum Analyselabor gefahren und dort vorzugsweise in eine Registrier- und Sortiermaschine entleert, welche die einzelnen Proben für die Analyse registriert und sortiert.

Optional kann der Transportbehälter eine innere Bodenfläche aufweisen und die verschließbare Oberseite mindestens so groß sein wie die innere Bodenfläche. Der Transportbehälter verjüngt sich vorzugsweise nicht nach oben hin zur Öffnung und weist vorzugsweise nach innen keine Vorsprünge oder Kanten auf. Dies ist vorteilhaft, um das Risiko eines gegenseitigen Blockierens der Proben beim Ausleeren in den Sammelbehälter deutlich zu verringern, bei dem unbemerkt nicht alle Proben in den Sammelbehälter durch die Öffnung fallen könnten.

Optional kann der Transportbehälter eine Aufnahmestruktur zur Aufnahme von diagnostischen Proben aufweisen, wobei die Aufnahmestruktur eine Mehrzahl von Aufnahmen aufweist, in denen eine Mehrzahl von diagnostischen Proben im Wesentlichen parallel zueinander in einer im Wesentlichen vertikalen Ausrichtung anordbar sind. Beispielsweise kann die Aufnahmestruktur eine Gitterstruktur oder ein Schaumstoffblock mit Aufnahmeöffnungen sein, in die Probenröhrchen von oben einsteckbar sind. Die Mehrzahl von Aufnahmen kann beispielsweise in einer übersichtlichen 5x6-Matrix angeordnet sein, sodass maximal 30 Proben im Transportbehälter von oben leicht zählbar angeordnet werden können.

Optional kann die verschließbare Oberseite des Transportbehälters vollständig öffenbar sein. Das heißt, dass sämtliche Seitwandungen des Transportbehälters direkt an die Oberseite angrenzen, die geöffnet vollständig als Öffnung ausgebildet ist.

Optional kann der Transportbehälter ein vorzugsweise zumindest teilweise transparentes Deckelelement aufweisen, das unverlierbar und beweglich am Transportbehälter befestigt ist und in einer Schließposition die Oberseite des Transportbehälters verschließt und in einer Öffnungsposition an einer Vorderwandung des Transportbehälters im Wesentlichen parallel zu dieser angeordnet ist. Die verschlossene Oberseite des Transportbehälters wird vorzugsweise durch das Deckelelement gebildet. Der Transportbehälter kann beispielsweise polyedrisch, vorzugsweise quaderförmig, ausgestaltet sein. Die Fläche der Vorderwandung kann im Wesentlichen der Fläche der Oberseite entsprechen. Das Deckelelement ist in der Öffnungsposition platzsparend außenseitig an der Vorderwandung verstaut. Es kann in der Öffnungsposition an der Vorderwandung anliegen oder in geringem Abstand davon angeordnet sein.

Optional kann das Deckelelement aus der Öffnungsposition um eine im Wesentlichen parallel zu einer von der Oberseite und der Vorderwandung gebildeten Vorderkante verlaufende Schwenkachse in eine im Wesentlichen horizontale Ausrichtung schwenkbar sein, aus welcher das Deckelelement im Wesentlichen horizontal in die Schließposition schiebbar ist.

Optional kann der Transportbehälter mindestens zwei innenseitige Nuten aufweisen, in denen das Deckelelement geführt ist, wobei die Nuten jeweils entlang einer Seitenkante verlaufen, die von der Oberseite und einer Seitenwandung des Transportbehälters gebildet sind. Das Deckelelement kann dazu seitlich mittels zwei Drehachsfortsätzen in die Nuten greifen, sodass die Drehachsfortsätze in der Öffnungsposition die Schwenkachse bilden und auf dem Weg in die Schließposition als Gleitschlitten in den Nuten fungieren.

Optional kann der Transportbehälter mindestens ein Verschlusselement mit einem vorderen festen Ende und einem elastisch verbiegbaren hinteren Ende aufweisen, wobei das hintere Ende in einen Bewegungspfad des Deckelelements von der Öffnungsposition in die Schließposition ragt, sodass das hintere Ende des Verschlusselements auf dem Weg in die Schließposition vom Deckelelement aus dem Bewegungspfad geschoben wird und bei Erreichen der Schließposition in eine Ausnehmung des Deckelelements verschließend einschnappt. Das Verschlusselement kann beispielsweise als vorzugsweise metallischer Federbügel ausgebildet sein, der sich im Wesentlichen vom vorderen festen Ende zum elastisch verbiegbaren hinteren Ende in einem relativ kleinen Winkel leicht nach unten vorgebogen verläuft. Das Verschlusselement ist vorzugsweise in vertikale Richtung wesentlich flexibler als in horizontale Richtung. Das hintere Ende bildet dadurch eine hintere Stoßkante, die in den Bewegungspfad des Deckelelements von der Öffnungsposition in die Schließposition ragt. Nach vorne hin bildet das hintere Ende dadurch eine schräge untere Gleitfläche, an der das Deckelelement auf dem Weg von der Öffnungsposition in die Schließposition entlang gleitet und damit das hintere Ende gegen die Rückstellkraft des Verschlusselements aus dem Bewegungspfad nach oben drückt. Die Ausnehmung des Deckelelements sorgt dafür, dass das hintere Ende durch die Vorspannung des Verschlusselements bei Erreichen der Schließposition darin verschließend nach unten einschnappen kann. Das Deckelelement kann dann nicht mehr ohne Weiteres zurückgeschoben werden, da das hintere Ende eine hintere Stoßkante bildet, die in die Ausnehmung des Deckelelements eingerastet ist, und das Verschlusselement in horizontale Richtung wenig flexibel ist.

Optional kann der Sammelbehälter mindestens ein magnetisches Öffnungselement an seiner oberseitigen Öffnung aufweisen, das derart angeordnet ist, dass es das hintere Ende des Verschlusselements aus der Ausnehmung des Deckelelements zieht, wenn sich der Transportbehälter in der Ausleerposition befindet. Vorzugsweise ist das Verschlusselement zumindest teilweise am hinteren Ende ferromagnetisch, sodass es vom Öffnungselement magnetisch angezogen wird, wenn es in der Ausleerposition nah genug daran positioniert ist. Bei mehreren Verschlusselementen ist vorzugsweise für jedes Verschlusselement ein Öffnungselement an definierter Stelle an der oberseitigen Öffnung des Sammelbehälters vorgesehen, um jedes Verschlusselement gleichzeitig zu öffnen, wenn sich der Transportbehälter in der Ausleerposition befindet.

Optional kann das Hilfsmittel Teil des Transportsystems sein, wobei das Hilfsmittel ein zumindest teilweise zum Transportbehälter korrespondierend geformtes Öffnungsmittel mit einem magnetischen Öffnungselement an einer definierten Position ist, und wobei das Öffnungsmittel derart definiert an der verschlossenen Oberseite des Transportbehälters platzierbar ist, dass die Oberseite dann auch öffenbar ist, wenn sich der Transportbehälter außerhalb der Ausleerposition befindet. Das Hilfsmittel kann beispielsweise in Form eines Griffs oder Bügels ausgestaltet sein, der definierte Anlageflächen aufweist, die nur in der definierten Position und Ausrichtung derart an dem Transportbehälter zur Anlage gebracht werden können, dass ein im Hilfsmittel angeordnetes magnetisches Öffnungselement so positioniert ist, dass es das hintere Ende des Verschlusselements aus der Ausnehmung des Deckelelements ziehen kann.

Optional kann die oberseitige Öffnung des Sammelbehälters an mindestens drei Seitenwandungen des Sammelbehälters angrenzen. Dadurch wird ein Ausleeren des Sammelbehälters besonders einfach und das Risiko reduziert, dass beim Ausleeren des Sammelbehälters versehentlich und unbemerkt Proben im Sammelbehälter verbleiben. Die Breite des Sammelbehälters entspricht vorzugsweise in etwa der Breite des Transportbehälters. Der Sammelbehälter hat dagegen vorzugsweise eine größere Tiefe und Höhe als der Transportbehälter.

Optional kann der Sammelbehälter mindestens drei Anlageflächen aufweisen, die sich in drei Anlageebenen erstrecken, welche einen gemeinsamen Schnittpunkt aufweisen, sodass die mindestens drei Anlageflächen die Ausleerposition eindeutig festlegen, in welcher der Transportbehälter umgedreht mit seiner Oberseite nach unten an den drei Anlageflächen anliegt. Vorzugsweise stehen die Anlageebenen in drei Dimensionen jeweils senkrecht aufeinander. Analog dazu kann das Hilfsmittel zueinander angeordnete Anlageflächen und Öffnungsmittel aufweisen, sodass der Transportbehälter außerhalb der Ausleerposition geöffnet werden kann, wenn das Hilfsmittel mit seinen Anlageflächen in definierter Position und Ausrichtung zur Anlage mit dem Transportbehälter kommt.

Gemäß einem zweiten Aspekt der vorliegenden Offenbarung wird ein Transportbehälter eines zuvor beschriebenen Transportsystems bereitgestellt. Der Transportbehälter kann beispielsweise als Ersatzteil bzw. als Erweiterung für ein vorhandenes Transportsystem dienen. Die verschlossene Oberseite des Transportbehälters ist dabei nur in der Ausleerposition ohne ein Hilfsmittel öffenbar und außerhalb der Ausleerposition nur mit dem Hilfsmittel öffenbar. Das Hilfsmittel kann für eine Mehrzahl von im Wesentlichen identisch ausgestalteten Transportbehältern zum Öffnen außerhalb der Ausleerposition verwendet werden. Alternativ dazu können verschiedene Transportbehälter in einem Transportsystem Verwendung finden.

Gemäß einem dritten Aspekt der vorliegenden Offenbarung wird ein Sammelbehälter eines zuvor beschriebenen Transportsystems bereitgestellt. Der Sammelbehälter kann beispielsweise als Ersatzteil bzw. als Erweiterung für ein vorhandenes Transportsystem dienen. Der Sammelbehälter definiert dabei die Ausleerposition, wobei die verschlossene Oberseite des Transportbehälters nur in der Ausleerposition ohne ein Hilfsmittel öffenbar ist und außerhalb der Ausleerposition nur mit dem Hilfsmittel öffenbar ist.

Gemäß einem vierten Aspekt der vorliegenden Offenbarung wird ein Verfahren zum Sammeln und Transportieren von diagnostischen Proben bereitgestellt mit den Schritten:
- Einsetzen einer Mehrzahl von diagnostischen Proben in eine Aufnahmestruktur in mindestens einem Transportbehälter durch eine verschließbare Oberseite des Transportbehälters, sodass die Mehrzahl von diagnostischen Proben im Wesentlichen parallel zueinander in einer im Wesentlichen vertikalen Ausrichtung im Transportbehälter angeordnet sind,
- Verschließen der Oberseite des Transportbehälters für den Transport des Transportbehälters mit einem Deckelelement, wobei die verschlossene Oberseite des Transportbehälters nur in einer Ausleerposition ohne ein Hilfsmittel öffenbar ist,
- Transportieren des Transportbehälters zu einem Sammelbehälter,
- Umdrehen des Transportbehälters zum Ausleeren der Mehrzahl von diagnostischen Proben in den Sammelbehälter mit verschlossener Oberseite nach unten,
- Platzieren des Transportbehälters auf eine oberseitige Öffnung des Sammelbehälters in die Ausleerposition, und
- Öffnen der Oberseite des Transportbehälters ohne das Hilfsmittel, sodass die Mehrzahl von diagnostischen Proben in den Sammelbehälter fällt.

Vorzugsweise dient das Deckelelement in der umgedrehten Ausleerposition des Transportbehälters kurzzeitig als Boden, auf den die Proben im Wesentlichen in Längsrichtung zumindest teilweise aus der Aufnahmestruktur fallen. In der Ausleerposition wird das kurzfristig als Boden dienende Deckelelement zum Öffnen weggezogen, sodass die Proben in den Sammelbehälter fallen können. Dies ist ohne das Hilfsmittel nur in der Ausleerposition möglich, da sonst das Deckelelement nicht weggezogen werden kann.

Optional kann das Verfahren ferner ein Bestimmen der Zahl der in dem mindestens einen Transportbehälter befindlichen Proben mittels Einsichtnahme durch das zumindest teilweise transparente Deckelelement und ein Quittieren des Transports der Zahl an Proben aufweisen. Vorzugsweise ist das Deckelelement beim Bestimmen der Zahl der in dem mindestens einen Transportbehälter befindlichen Proben verschlossen. Im Falle eines nicht teilweise transparente Deckelelements kann das Deckelelement beim Bestimmen der Zahl der in dem mindestens einen Transportbehälter befindlichen Proben geöffnet sein und erst mit dem Quittieren verschlossen werden.

Die Offenbarung ist nachfolgend anhand von in den Zeichnungen dargestellten Ausführungsbeispielen näher erläutert. Es zeigen:
Fig. 1 ein Ausführungsbeispiel des hierin offenbarten Transportsystems mit geöffnetem Sammelbehälter und zwei geöffneten Transportbehältern;
Fig. 2 ein Ausführungsbeispiel eines hierin offenbarten Transportsystems mit einem geschlossenen Sammelbehälter und einem geschlossenen Transportbehälter;
Fig. 3 ein Ausführungsbeispiel eines hierin offenbarten Transportsystems mit einem geöffneten Sammelbehälter (ohne linke Seitenwand) und einem geöffneten Transportbehälter in der Ausleerposition;
Fig. 4 eine Seitenansicht des in Fig. 3 dargestellten Ausführungsbeispiels;
Fig. 5 ein Ausführungsbeispiel eines hierin offenbarten Transportsystems mit einem geöffneten Sammelbehälter und einem geschlossenen Transportbehälter in der Ausleerposition;
Fig. 6 ein Ausführungsbeispiel eines hierin offenbarten Transportsystems mit einem geöffneten Sammelbehälter und einem geöffneten Transportbehälter in der Ausleerposition;
Fig. 7 das in Fig. 5 gezeigte Ausführungsbeispiel in einer Vorderansicht (rechts unten), in einer Schnittansicht durch die Ebene A-A (unten links) und einer Detailansicht C (oben);
Fig. 8 das in Fig. 7 gezeigte Ausführungsbeispiel mit einem geschlossenen Transportbehälter in der Ausleerposition in einer Vorderansicht wie in Fig. 7, aber mit einer Schnittansicht durch eine andere Schnittebene A-A und einer Detailansicht C auf das Verschlusselement;
Fig. 9 die gleichen Ansichten wie in Fig. 8 mit einem geschlossenen Transportbehälter außerhalb der Ausleerposition;
Fig. 10 ein Ausführungsbeispiel eines hierin offenbarten Transportbehälters in einer Vorderansicht (unten) und einer Schnittansicht durch die Ebene A-A (oben), wobei das hintere Ende des Verschlusselements in die Ausnehmung des Deckelelements verschließend eingeschnappt ist (außerhalb der Ausleerposition);
Fig. 11 das Ausführungsbeispiel gemäß Fig. 10, wobei das hintere Ende des Verschlusselements aus dem Bewegungspfad des Deckelements gezogen ist (in der Ausleerposition);
Fig. 12 ein Ausführungsbeispiel des hierin offenbarten Transportbehälters in einer Oberansicht (oben links), einer Schnittansicht durch die Ebene A-A (unten links) und einer Detailansicht C (rechts), wobei sich das Deckelelement des Transportbehälters in einer Öffnungsposition befindet;
Fig. 13 ein Ausführungsbeispiel eines hierin offenbarten geschlossenen Sammelbehälters in einer Hinteransicht (links) und einer Schnittansicht durch die Ebene A-A (rechts);

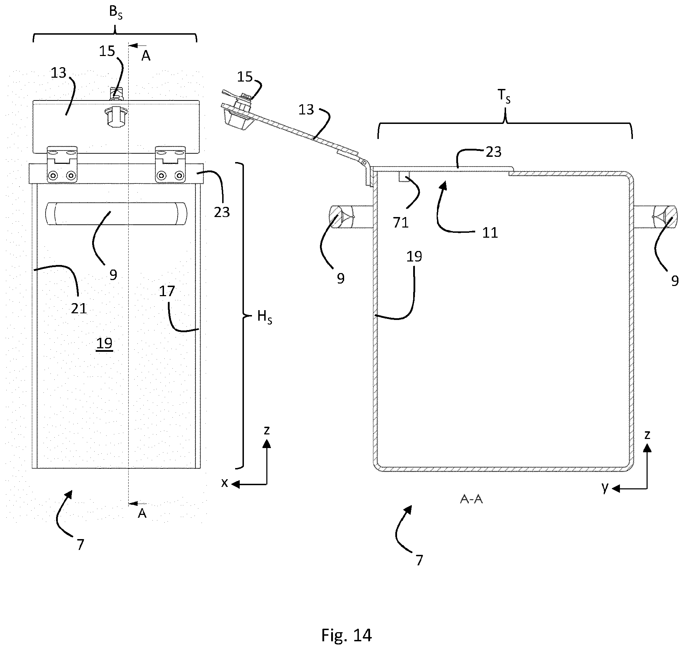

Fig. 14 ein Ausführungsbeispiel eines hierin offenbarten geöffneten Sammelbehälters in einer Hinteransicht (links) und einer Schnittansicht durch die Ebene A-A (rechts);
Fig. 15 ein Ausführungsbeispiel des hierin offenbarten Transportbehälters in einer perspektivischen Ansicht (oben), einer Vorderansicht (links unten) und einer Schnittansicht durch die Ebene A-A (unten rechts), wobei ein Hilfsmittel zum Öffnen des Transportbehälters am Transportbehälter angesetzt ist; und
Fig. 16 das Hilfsmittel aus Fig. 15 in einer perspektivischen Ansicht (oben), einer Vorderansicht (Mitte) und einer Unteransicht (unten).

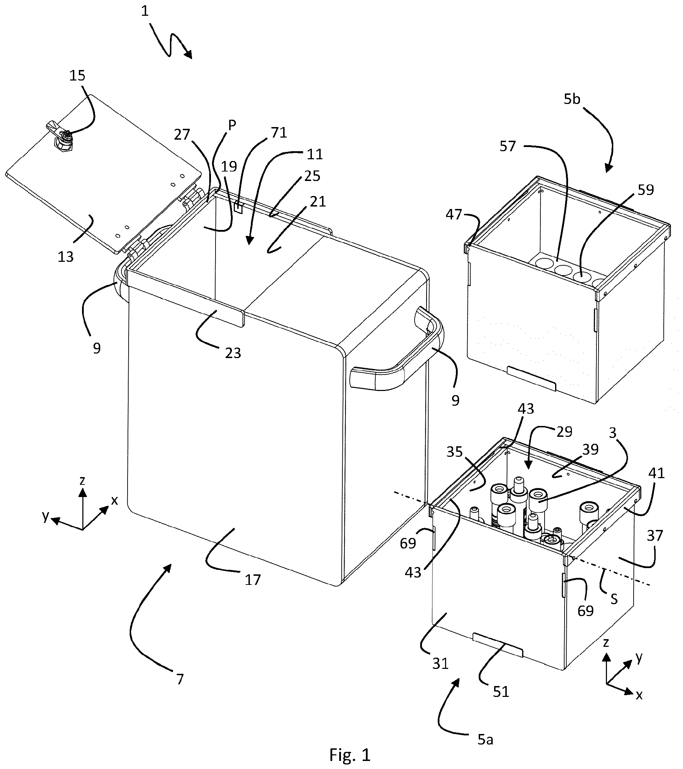

Fig. 1 zeigt ein Transportsystem 1 für diagnostische Proben 3, die sich in einem ersten mobilen Transportbehälter 5a befinden. Zum Sammeln und Transportieren weiterer Proben 3 weist das Transportsystem 1 zusätzlich einen oder mehrere weitere im Wesentlichen zum ersten Transportbehälter 5a identische mobile Transportbehälter 5b auf. Außerdem weist das Transportsystem 1 einen Sammelbehälter 7 auf, in den die mobilen Transportbehälter 5a, 5b entleert werden können.

Zum besseren Verständnis weisen die Figuren jeweils ein rechtshändiges kartesisches Koordinatensystem auf, wobei die z-Richtung der Vertikalen nach oben, die x-Richtung eine horizontale Richtung in der Breite und die y-Richtung eine horizontale Richtung in der Tiefe entspricht. Entsprechend sind hier Begriffe wie "oben", "unten", "vorne", "hinten", "links" und rechts" im Sinne des zugehörigen angezeigten Koordinatensystems mit Blickrichtung auf den Gegenstand in y-Richtung zu verstehen.

Der Sammelbehälter 7 kann sich typischerweise in einem Tramsportfahrzeug eines Kurierdienstes befinden und mit den Proben 3 aus einer Vielzahl von Transportbehältern 5a, 5b befüllt werden. Die Transportbehälter 5a, 5b können sich beispielsweise in einem Krankenhaus oder einer Arztpraxis befinden und dort mit den Proben 3 befüllt werden. Bei Abholung der Proben 3 durch einen Kurierdienst werden die gefüllten Transportbehälter 5a, 5b verschlossen und vom Kurierdienst zum Sammelbehälter 7 gebracht, wo sie in den Sammelbehälter 7 ausgeleert werden und leer wieder zurück zum Krankenhaus oder zur Arztpraxis gebracht werden. Der gefüllte Sammelbehälter 7 wird verschlossen zu einem Analyselabor gefahren, wo er beispielsweise in eine Registrier- und Sortiermaschine ausgeschüttet werden kann, so dass die als Schüttgut in dem Sammelbehälter 7 befindlichen Proben 3 im Labor untersucht werden können. Der Sammelbehälter 7 ist also ebenfalls mobil für die Wegstrecke aus dem Transportfahrzeug zum Analyselabor. Da das Gewicht eines gefüllten Sammelbehälters 7 durchaus beträchtlich sein kann, weist der Sammelbehälter 7 vorderseitig sowie rückseitig jeweils einen Tragegriff 9 zum besseren Handling auf.

Der Sammelbehälter 7 weist außerdem eine oberseitige Öffnung 11 auf, durch die der Sammelbehälter sowohl befüllt als auch entleert werden kann. Die oberseitige Öffnung 11 ist durch einen Verschlussdeckel 13 verschließbar, welcher rückseitig oben am Sammelbehälter 7 angelenkt ist. Der Verschlussdeckel 13 weist dazu einen Verschluss 15 auf, mit dem bei geschlossener Stellung des Verschlussdeckels 13 die oberseitige Öffnung 11 derart verschließbar ist, dass der Sammelbehälter 7 ohne einen Schlüssel nicht zerstörungsfrei geöffnet werden kann.

Die oberseitige Öffnung 11 des Sammelbehälters 7 grenzt an drei Seitenwandungen des Sammelbehälters 7 an, nämlich an der linken Seitenwand 17, an der Rückwand 19 sowie an der rechten Seitenwand 21. An diesen Seitenwandungen 17, 19, 21 sind Anlageleisten 23 angeordnet, die die oberseitige Öffnung 11 zumindest teilweise einfassen und nach innen Anlageflächen 25 bilden, die mit einer oberseitigen Stirnfläche 27 der Seitenwandungen 17, 19, 21 des Sammelbehälters 7 eine Ausleerposition für die Transportbehälter 5a, 5b eindeutig definieren.

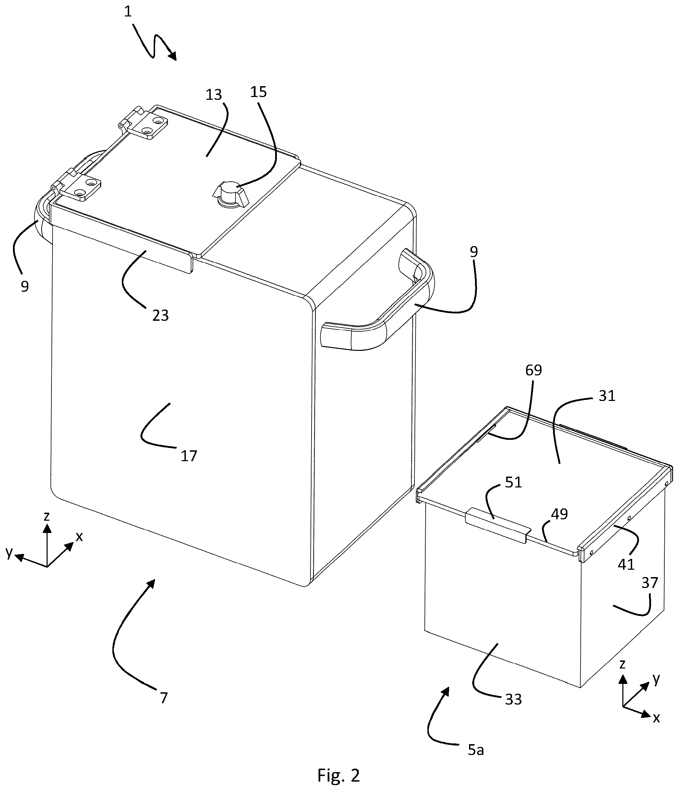

Die Transportbehälter 5a, 5b weisen ebenfalls eine verschließbare Oberseite 29 auf, wobei die verschließbare Oberseite 29 in etwa in Form und Größe der oberseitigen Öffnung 11 des Sammelbehälters entspricht. Die verschließbare Oberseite 29 der Transportbehälter 5a, 5b grenzt an alle vier Seitenwandungen des Transportbehälters 5a, 5b an, so dass die Oberseite 29 in dem in Fig. 1 gezeigten geöffneten Zustand vollständig als Öffnung ausgebildet ist. Zum Verschließen des Transportbehälters 5a, 5b weist dieser ein transparentes Deckelelement 31 auf, das unverlierbar und beweglich am Transportbehälter 7 befestigt ist und in einer Schließposition (siehe Fig. 2) die Oberseite 29 des Transportbehälters 5a, 5b verschließt und in einer Öffnungsposition (siehe Fig. 1) an einer Vorderwand 33 des Transportbehälters im Wesentlichen parallel zu dieser angeordnet ist. Ähnlich zum Sammelbehälter 7 ist die verschließbare Oberseite 29 des Transportbehälters 5a, 5b an drei Seitenwandungen, nämlich einer linken Seitenwand 35, einer rechten Seitenwand 37 und einer Rückwand 39 von Leisten 41 umfasst. Die Leisten 41 bilden an den Seitenwänden 35, 37 innenseitige Nuten 43 aus, in denen das Deckelelement 31 geführt ist. Die Nuten 43 verlaufen dabei jeweils horizontal in y-Richtung entlang einer Seitenkante 45, die von der Oberseite 29 und der jeweiligen Seitenwand 35, 37 des Transportbehälters 5a, 5b gebildet sind. Das Deckelelement 31 weist dazu seitlich zwei Drehachsfortsätze 47 auf, die jeweils in die Nuten 43 greifen, so dass die Drehachsfortsätze 47 in der Öffnungsposition (siehe Fig. 1) eine Schwenkachse S bilden und auf dem Weg in die Schließposition (siehe Fig. 2) als Gleitschlitten in den Nuten 43 fungieren. Die Schwenkachse S verläuft im Wesentlichen parallel zu einer von der Oberseite 29 und der Vorderwand 33 gebildeten vorderen Oberkante 50 des Transportbehälters 5a, 5b und relativ nah zu dieser.

Die Größe der Vorderwand 33 des Transportbehälters 5a, 5b entspricht in etwa der Größe des Deckelelements 31. Dadurch kann das Deckelelement 31 platzsparend in der Öffnungsposition (siehe Fig. 1) an der Vorderwand 33 angeordnet sein. Das Deckelelement 31 weist an einer Vorderkante 49, welche in der Öffnungsposition (siehe Fig. 1) nach unten zeigt, ein Griffelement 51 auf. Das Griffelement 51 umgreift L-förmig die Vorderkante 49 des Deckelements 31 und klemmt sich mit einer Grifffläche in der Öffnungsposition unter einem Boden 53 des Transportbehälters fest. Damit ist das Deckelement 31 in der Öffnungsposition stabil gelagert. Zum Schließen des Transportbehälters 5a, 5b kann der Transportbehälter vorne angehoben werden und das Griffelement 51 mit den Fingern nach vorn aus der Verklemmung mit dem Boden 53 gezogen werden. Dabei wird das Deckelement 31 um die Schwenkachse S um 90° nach oben geschwenkt, um dann parallel zu den Nuten 43 in die Schließposition (siehe Fig. 2) horizontal in y-Richtung nach hinten geschoben zu werden.

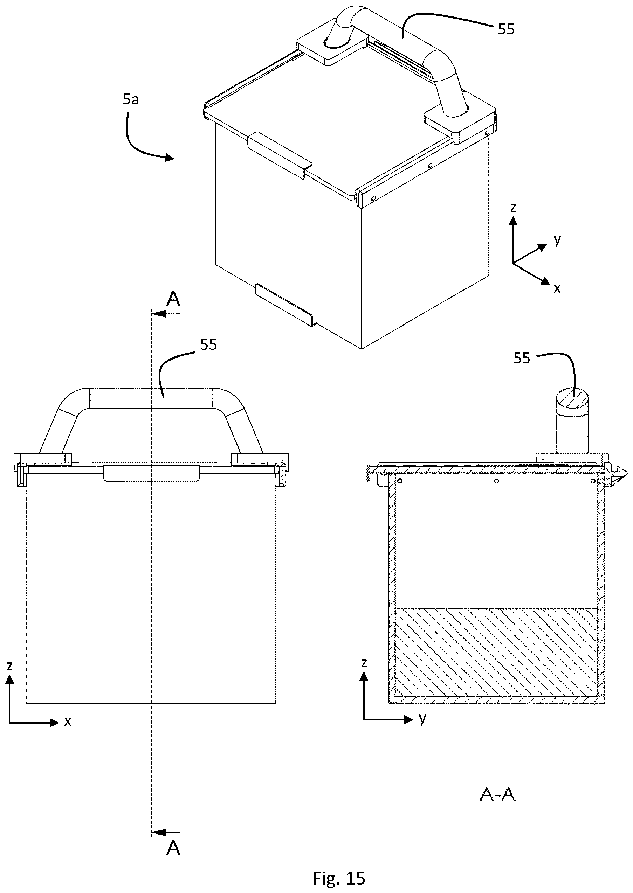

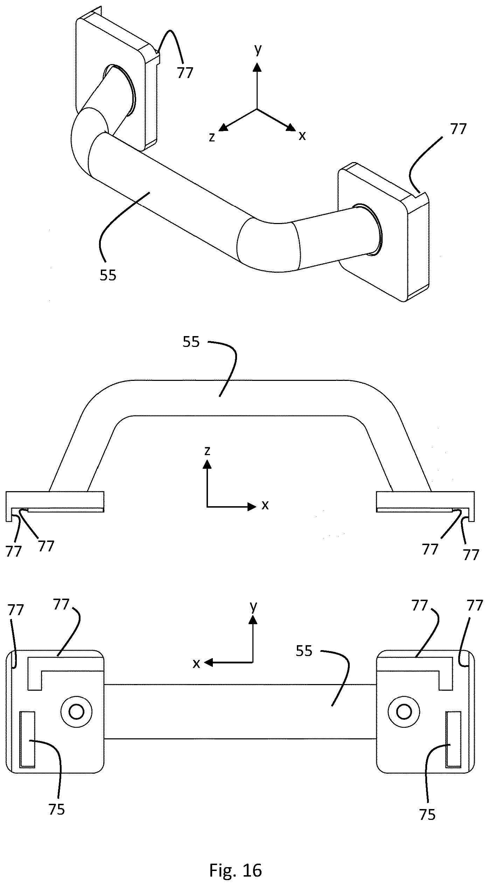

In Fig. 2 sind sowohl der Sammelbehälter 7 als auch der Transportbehälter 5a verschlossen gezeigt. Der Sammelbehälter 7 ist mit einem für den Verschluss 15 passenden Schlüssel öffenbar, wobei der Verschlussdeckel 13 um mehr als 90° nach hinten geschwenkt werden kann. Der Transportbehälter 5a ist hingegen nicht mehr ohne Weiteres öffenbar, wenn sich das Deckelelement 31 in der Schließposition (siehe Fig. 2) befindet. Es gibt nur zwei Möglichkeiten, den Transportbehälter 5a zerstörungsfrei zu öffnen. Die bei normaler Verwendung des Transportsystems vorgesehene Möglichkeit ist die, den Transportbehälter 5a in die von dem Sammelbehälter 7 definierte Ausleerposition zu bringen. Die zweite Möglichkeit, die nur in Ausnahmefällen von autorisierten Personen durchgeführt werden sollte, ist eine Verwendung eines in Fig. 15 und 16 gezeigten Hilfsmittels 55. Das Hilfsmittel 55 steht nur autorisierten Personen, wie etwa Personal des Krankenhauses oder der Arztpraxis zur Verfügung. Für einen Kurier gibt es nur die Möglichkeit, den Transportbehälter 5a zu öffnen, indem er ihn in die Ausleerposition bringt.

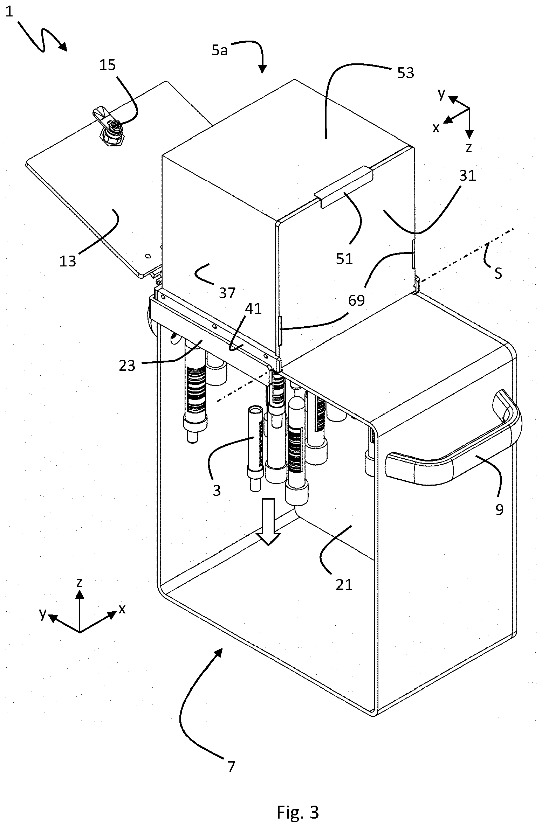

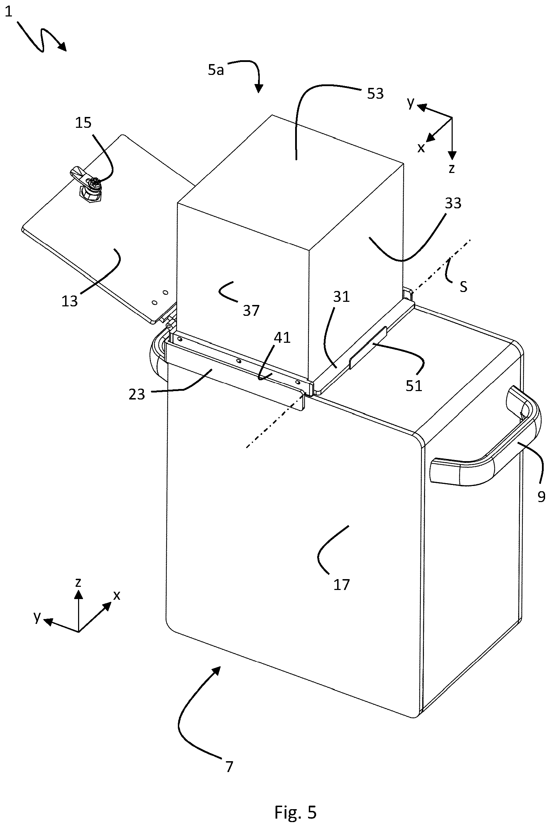

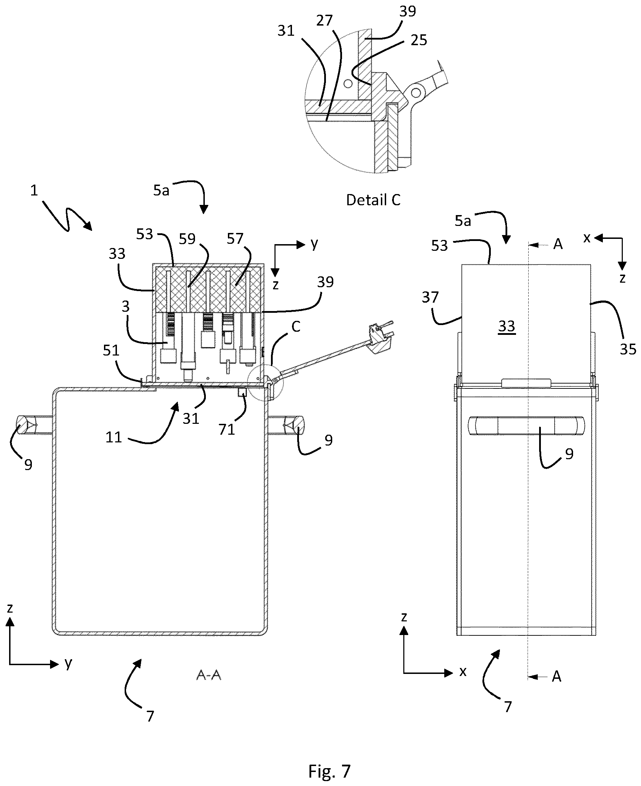

Fig. 3 zeigt den Transportbehälter 5a in geöffneter Stellung in der Ausleerposition über der verschließbaren Öffnung 11 des Sammelbehälters 7. In Fig. 3 ist die linke Seitenwand 17 des Sammelbehälters 7 nicht gezeigt, um besser darzustellen, wie die Proben 3 beim Ausleeren des Transportbehälters 5a in den Sammelbehälter 7 fallen. Um den Transportbehälter 5a in die in Fig. 3 gezeigte Ausleerposition zu bringen, wurde er zuvor im geschlossenen Zustand umgedreht, so dass das Deckelelement 31 kurzzeitig als Boden dient (siehe Fig. 5 und 7).

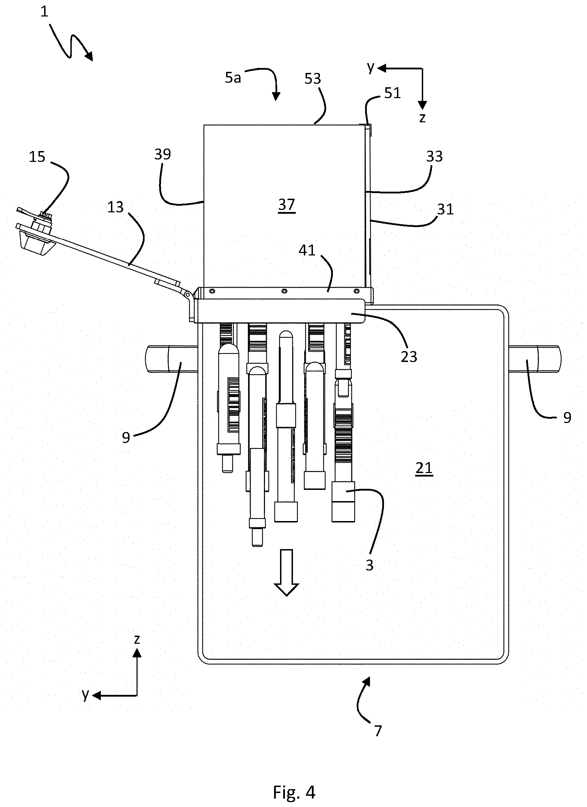

In den Fig. 3 und 4 wird ein besonderer Vorteil der gezeigten Ausführungsform deutlich, da die Proben 3 relativ geordnet und mit vertikaler Ausrichtung aus dem Transportbehälter 5a in den Sammelbehälter 7 fallen. Der Transportbehälter 5a, 5b weist nämlich eine Aufnahmestruktur 57 auf, beispielsweise in Form eines Schaumstoffblocks, wobei die Aufnahmestruktur 57 eine Mehrzahl von Aufnahmen 59 aufweist, hier als Einstecklöcher im Schaumstoffblock 57 gezeigt, in denen die Proben 3 im Wesentlichen parallel zueinander in einer im Wesentlichen vertikalen Ausrichtung anordbar sind. Die Proben 3 werden lediglich von oben in die Aufnahmen 59 gesteckt und können entsprechend bei umgedrehtem Transportbehälter 5a aus diesen vertikal herausfallen, wenn sich der Transportbehälter 5a in der Ausleerposition befindet (siehe Fig. 3 und 4). Vorzugsweise sind die Aufnahmen mindestens so tief wie der Abstand der kürzesten Probe 3 vom verschlossenen Deckelement 31, so dass selbst die kürzesten Proben 3 in der in Fig. 5 und 7 gezeigten umgedrehten, aber noch verschlossenen Stellung des Transportbehälters 5a zumindest noch teilweise in der zugehörigen Aufnahme 59 stecken. Dadurch behalten die Proben 3 in jedem Fall beim verschlossenen Transportbehälter 5a ihre Ordnung.

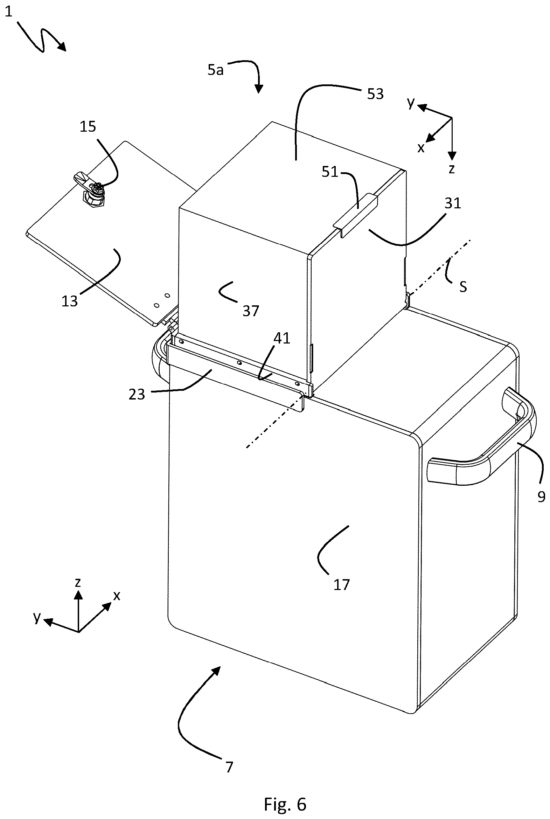

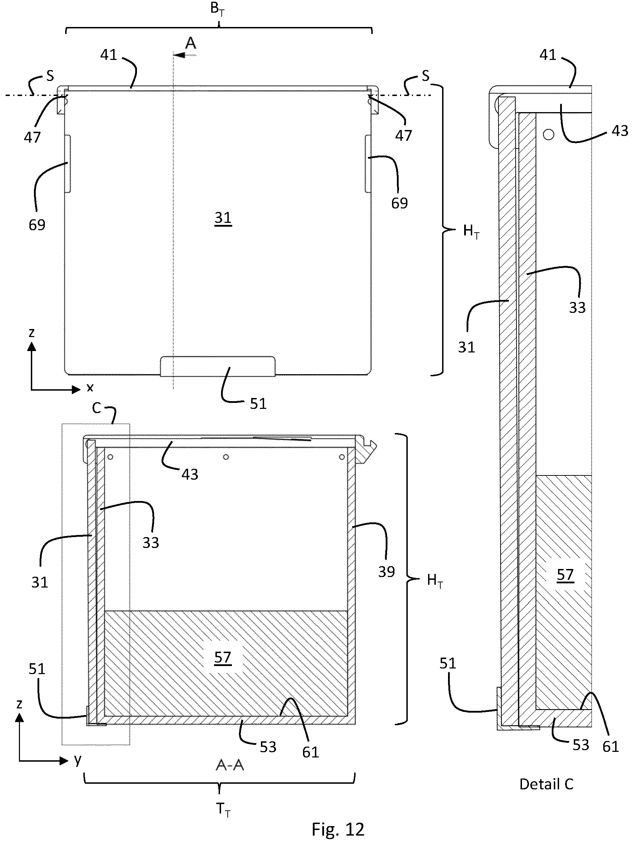

In Fig. 6 ist der Transportbehälter 5a geöffnet, wobei das Deckelelement 31 horizontal nach vorne (in negative y-Richtung) gezogen und anschließend um die Schwenkachse S um 90° nach oben in die Öffnungsposition geschwenkt wurde. Sobald die Proben 3 in den Sammelbehälter 7 gefallen sind, kann der Transportbehälter 5a wieder aus der Ausleerposition genommen werden und zur weiteren Befüllung mit Proben 3 zurück ins Krankenhaus oder die Arztpraxis gebracht werden. Fig. 7 zeigt die in Fig. 5 gezeigte Situation detailliert im Längsschnitt entlang der Ebene A-A. Es wird deutlich, dass die oberseitige Öffnung 11 des Sammelbehälters ein klein wenig größer ist als die verschließbare Oberseite 29 des Transportbehälters. Beim Übergang zwischen Transportbehälter 5a und Sammelbehälter 7 gibt es keine Verjüngung oder Vorsprünge bzw. Kanten nach innen, an denen sich die Proben 3 verkanten könnten. Damit ist das Risiko eines gegenseitigen Blockierens der Proben 3 beim Ausleeren in den Sammelbehälter 7 deutlich verringert. Die polyedrische, im Wesentlichen quaderförmige, Ausgestaltung sowohl des Sammelbehälters 7 als auch des Transportbehälters 5a unterstützt diesen Zweck. Der Boden 53 des Transportbehälters 5a bildet eine Bodenfläche 61 (siehe Fig. 12), die im Wesentlichen genau so groß ist, wie die vollständig öffenbare Oberseite 29. Eine kleinere innere Bodenfläche 61 wäre denkbar, wobei sich das Innenvolumen des Transportbehälters 5a nach oben hin aufweiten würde. Allerdings wäre eine größere innere Bodenfläche 61 nachteilhaft, da sich das Innenvolumen des Transportbehälters 5a nach oben hin verjüngen würde, was ein gegenseitiges Blockieren der Proben begünstigte.

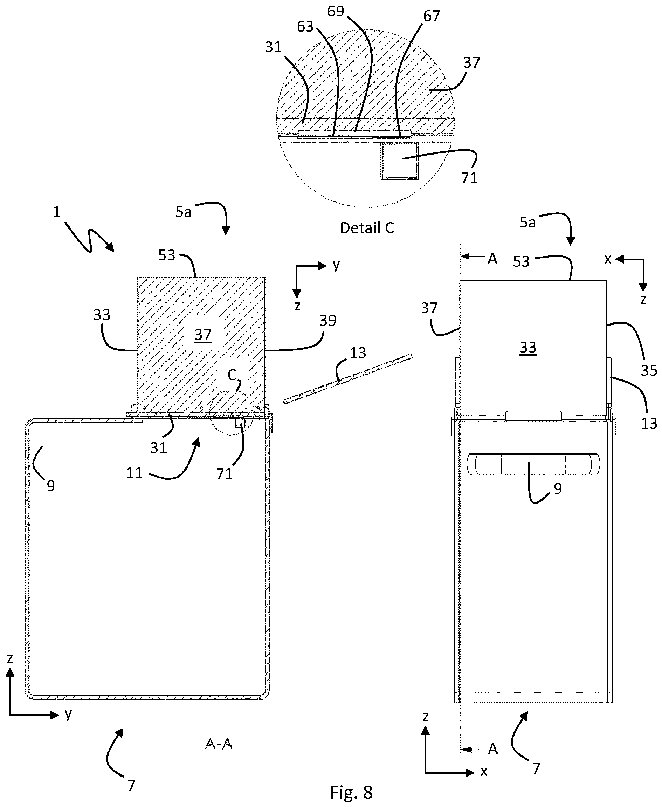

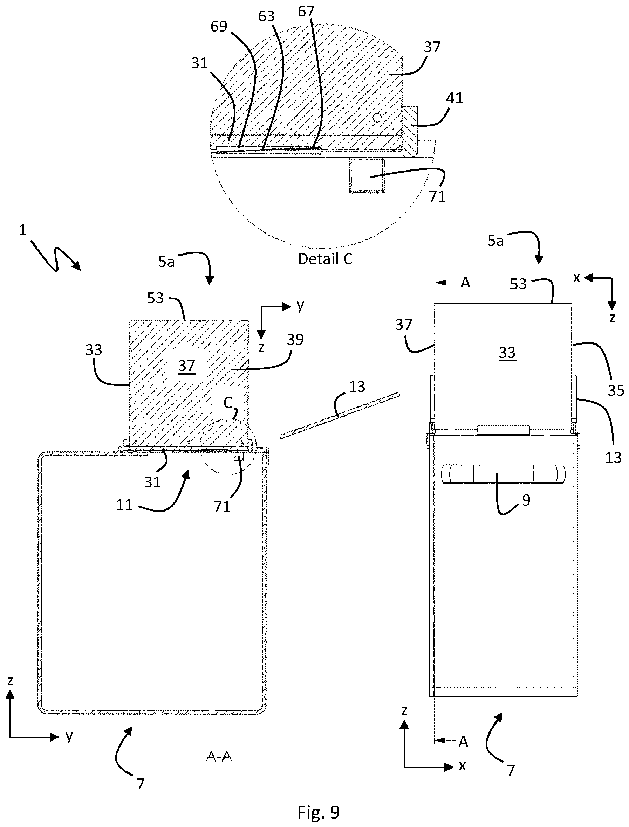

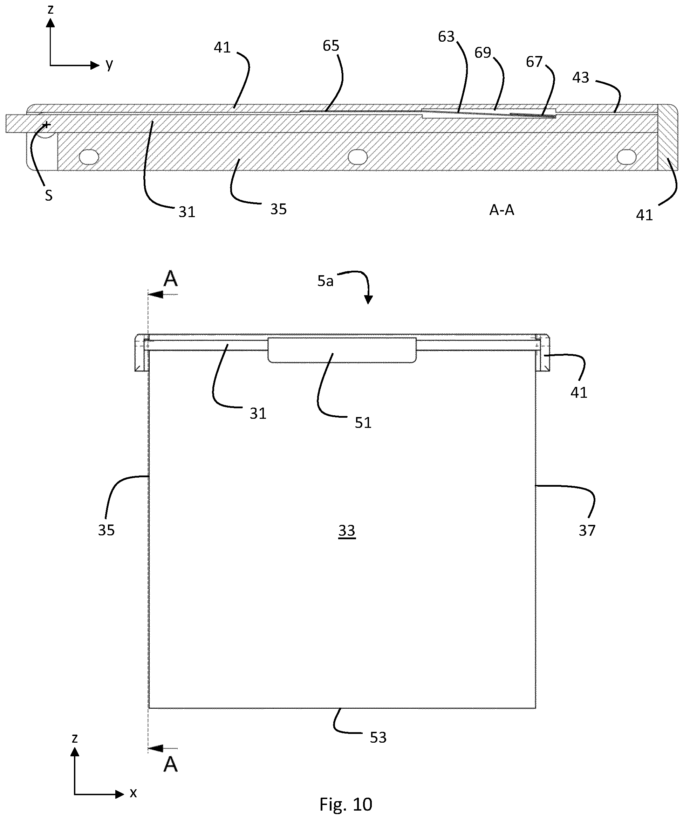

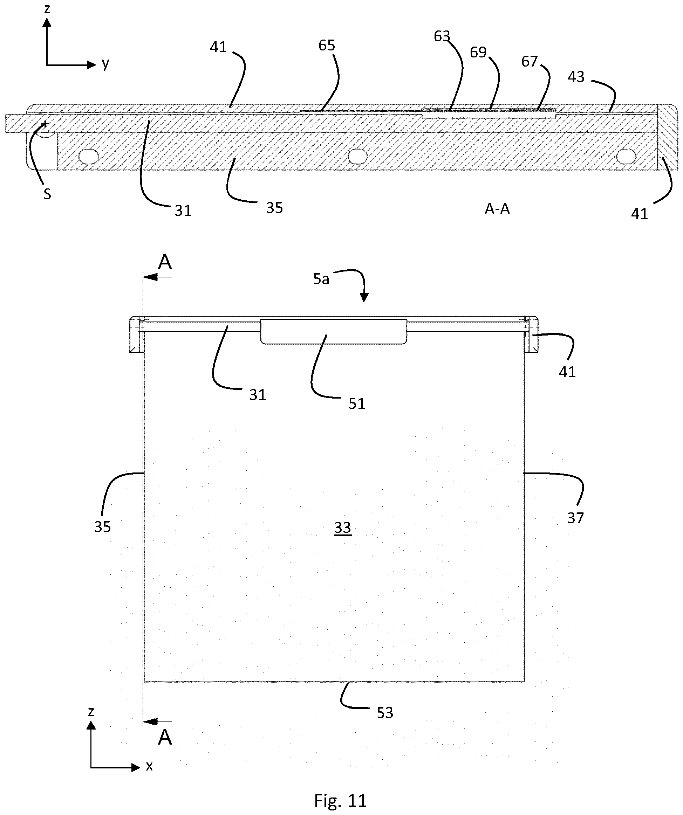

Fig. 8 bis 11 verdeutlichen die Funktion wie der Transportbehälter 5a verschlossen bzw. geöffnet wird. In Fig. 8 befindet sich der Transportbehälter 5a in der Ausleerposition umgedreht mit verschlossener Oberseite 29 nach unten auf der oberseitigen Öffnung 11 des Sammelbehälters 7. In den seitlichen Leisten 41 des Transportbehälters 5a ist jeweils ein Verschlusselement 63 in Form eines länglichen Federbügels angeordnet. Das Verschlusselement 63 erstreckt sich im Wesentlichen in y-Richtung entlang eines Abschnitts der jeweiligen Nut 43, wobei ein vorderes festes Ende 65 oberhalb der Nut 43 befestigt ist und ein elastisch verbiegbares hinteres Ende 67 in einem leichten Bogen in die Nut 43, also in den Bewegungspfad des Deckelelements 31 (siehe Fig. 9 und 10) ragt. Das Deckelelement 31 weist an den beiden Seitenkanten oberseitige Ausnehmungen 69 auf, in welche das hintere Ende 67 des Verschlusselementes 63 bei Erreichen der Schließposition des Deckelelements 31 verschließend einschnappt. Das hintere Ende 67 des Verschlusselements 63 ist relativ einfach in vertikale z-Richtung verbiegbar, allerdings ist das Verschlusselement 63 in horizontaler y-Richtung wenig flexibel. Sobald also das hintere Ende 67 in die oberseitige Ausnehmung 69 des Deckelelements 31 eingeschnappt ist, ist das Deckelelement 31 nicht mehr ohne Weiteres nach vorne hin (in negative y-Richtung) öffenbar. Das Verschlusselement 63 ist so mit seinem hintere Ende 67 nach unten hin vorgespannt, dass das hintere Ende 67 nicht ohne Weiteres aus der oberseitigen Ausnehmung 69 des Deckelements zu bringen ist.

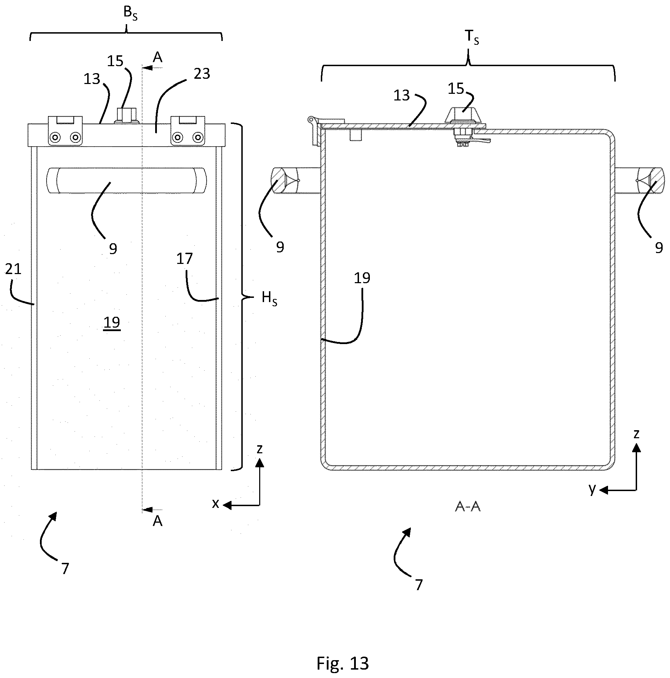

Zum Öffnen des Transportbehälters 5a in der Ausleerposition weist der Sammelbehälter 7 oben an den Seitenwänden 17 und 21 genau positionierte magnetische Öffnungselemente 71 auf. Die magnetischen Öffnungselemente 71 sind derart angeordnet, dass sie das hintere Ende 67 des Verschlusselements 63 dann und nur dann aus der Ausnehmung 69 des Deckelelements 31 ziehen, wenn sich der Transportbehälter 5a in der Ausleerposition befindet. Um den Transportbehälter 5a in einfacher Weise in die Ausleerposition bringen zu können, bilden die Anlageleisten 23 und die oberseitigen Stirnflächen 27 der Seitanwandungen 35, 37, 39 die Anlageflächen 25, die einen gemeinsamen Schnittpunkt P (s. Fig. 1) an den hinteren beiden Ecken der Öffnung 11 aufweisen, so dass die Anlageflächen 25 die Ausleerposition eindeutig festlegen, in welcher der umgedrehte Transportbehälter 5a mit seinen Leisten 41 an den Anlageflächen 25 anliegt. Der Transportbehälter 5a muss also nur mit seiner Oberseite 29 nach unten zwischen die Anlageleisten 23 des Sammelbehälter 7 positioniert werden und ganz bis zum Anschlag an die hintere Anlagefläche 25 geschoben werden (siehe Fig. 8). In dieser Ausleerposition ist das hintere Ende 67 des Verschlusselements 63 der maximalen magnetischen Anziehungskraft des magnetischen Öffnungselements 71 ausgesetzt, da es den geringsten Abstand zum magnetischen Öffnungselement 71 hat. In der Ausleerposition ist der Einfluss des magnetischen Feldes des Öffnungselements 71 ausreichend, um das hintere Ende 67 des Verschlusselements 63 in eine Ausnehmung 73 in der Nut zu ziehen, so dass das Verschlusselement 63 im Wesentlichen gerade verläuft und das hintere Ende 67 des Verschlusselements 63 nicht mehr in den Bewegungs- bzw. die Ausnehmung 69 des Deckelements 31 ragt. In der in Fig. 8 gezeigten Ausleerposition ist es also möglich, mit den Fingern die Griffleiste 51 nach vorne in negative y-Richtung zu ziehen und damit das Deckelelement 31 zu öffnen. Da das Deckelelement 31 in der noch geschlossenen Ausleerposition (siehe Fig. 5 und 7) kurzzeitig als Boden fungiert und somit die Proben 3 von Innen auf dem Deckelelement 31 stehen, fallen sie durch die oberseitige Öffnung 11 in den Sammelbehälter 7, wenn das Deckelelement 31 vollständig nach vorne weggezogen wird. Damit das Deckelelement 31 dazu genügend Platz hat, hat der Sammelbehälter 7 vorzugsweise eine mindestens doppelt so große Tiefe Ts (in y-Richtung) wie die oberseitige Öffnung 11 (siehe Fig. 13). Die Breite Bₛ des Sammelbehälters 7 entspricht im Wesentlichen der Breite B_{T} des Transportbehälters 5a. Die Höhe Hs (in z-Richtung) des Sammelbehälters 7 sollte mindestens so groß sein, dass eine Vielzahl von Transportbehältern 5a darin ausgeleert werden können. Allerdings sollte der Sammelbehälter 7 nicht so hoch sein, dass er bei voller Beladung zu schwer zum Tragen wäre. Es hat sich herausgestellt, dass es vorteilhaft ist, den Sammelbehälter 7 in etwa so hoch wie tief und etwa halb so breit auszugestalten. Die Transportbehälter 5a sind dann im Wesentlichen fast würfelförmig, also B_{T} ≈ T_{T}≈ H_{T}, mit einem Volumen, das im Wesentlichen einem Viertel des Sammelbehälters 7 entspricht. Das Fassungsvermögen des Sammelbehälters 7 im Hinblick auf die Anzahl der Proben 3 ist allerdings sehr viel höher, da die Proben 3 im Sammelbehälter 7 als Schüttgut gesammelt sind und nicht wie in den Transportbehältern 5a, 5b mittels einer Aufnahmestruktur geordnet. Es können also ohne Weiteres mehr als 20 Transportbehälter 5a, 5b in den Sammelbehälter 7 umgefüllt werden.

In Fig. 15 und 16 ist die Öffnung des Transportbehälters 5a mittels eines Hilfsmittels 55 außerhalb der Ausleerposition gezeigt. Das Hilfsmittel 55 ist ein vom Transportbehälter 5a, 5b separates Element in Form eines Bügels, der zumindest teilweise zum Transportbehälter 5a korrespondierend geformt und ähnlich dem Sammelbehälter 7 magnetische Öffnungselemente 75 an definierten Positionen aufweist. Das Öffnungsmittel 55 ist derart definiert an der verschlossenen Oberseite 29 des Transportbehälters 5a, 5b platzierbar, dass die Oberseite 29 ausnahmsweise auch dann öffenbar ist, wenn sich der Transportbehälter 5a, 5b außerhalb der Ausleerposition befindet. Das Öffnungsmittel 55 weist ähnlich zum Sammelbehälter 7 Anlageflächen 77 auf, die bei Anlage am Transportbehälter 5a genau eine Positionierung des Öffnungsmittels 55 relativ zum Transportbehälter 5a definieren, bei der die magnetischen Öffnungsmittel 75 so nah am hinteren Ende 67 des Verschlusselements 63 liegen, dass dieses aus der Ausnehmung 69 des Deckelelements 31 gezogen wird. Dadurch ist mit Hilfe des Öffnungsmittels 55 der Transportbehälter 5a ausnahmsweise auch außerhalb der Ausleerposition öffenbar. Diese Möglichkeit steht allerdings nur autorisierten Personen zur Verfügung, die über das Hilfsmittel 55 verfügen.

Das hierin beschriebene Transportsystem ist sicherer und einfacher zu handhaben als bekannte Transportsysteme dieser Art.

Die nummerierten Bezeichnungen der Bauteile oder Bewegungsrichtungen als "erste", "zweite", "dritte" usw. sind hierin rein willkürlich zur Unterscheidung der Bauteile oder Bewegungsrichtungen untereinander gewählt und können beliebig anders gewählt werden. Es ist damit kein Bedeutungsrang verbunden. Eine Bezeichnung eines Bauteils oder technischen Merkmals als "erstes" soll nicht dahingehend missverstanden werden, dass es ein zweites Bauteil oder technisches Merkmal dieser Art geben muss. Außerdem können etwaige Verfahrensschritte, soweit nicht explizit anders erläutert oder zwingend erforderlich, in beliebiger Reihenfolge und/oder zeitlich teilweise oder ganz überlappend durchgeführt werden.

Äquivalente Ausführungsformen der hierin beschriebenen Parameter, Bauteile oder Funktionen, die in Anbetracht dieser Beschreibung einer fachlich versierten Person als offensichtlich erscheinen, seien hierin so erfasst als wären sie explizit beschrieben, sofern sie nicht vom Gegenstand der Ansprüche abweichen. Als optional, vorteilhaft, bevorzugt, erwünscht oder ähnlich bezeichnete "kann"-Merkmale sind als optional zu verstehen und nicht als schutzbereichsbeschränkend.

Die beschriebenen Ausführungsformen sind als illustrative Beispiele zu verstehen und stellen keine abschließende Liste von möglichen Ausführungsformen dar. Im Übrigen soll hierin weder der Begriff "aufweisen" zusätzliche andere Merkmale oder Verfahrensschritte ausschließen noch soll "ein" oder "eine" eine Mehrzahl ausschließen.

### Bezugszeichenliste:

- 1: Transportsystem
- 3: Proben
- 5a, 5b: Transportbehälter
- 7: Sammelbehälter
- 9: Griffe
- 11: oberseitige Öffnung des Sammelbehälters
- 13: Verschlussdeckel des Sammelbehälters
- 15: Verschluss des Deckelements des Sammelbehälters
- 17: linke Seitenwand des Sammelbehälters
- 19: Rückwand des Sammelbehälters
- 21: rechte Seitenwand des Sammelbehälters
- 23: Anlageleisten des Sammelbehälters
- 25: Anlageflächen des Sammelbehälters
- 27: obere Seitenkanten des Sammelbehälters
- 29: verschließbare Oberseite des Transportbehälters
- 31: Deckelelement
- 33: Vorderwand des Transportbehälters
- 35: linke Seitenwand des Transportbehälters
- 37: rechte Seitenwand des Transportbehälters
- 39: Rückwand des Transportbehälters
- 41: Leisten des Transportbehälters
- 43: Nuten des Transportbehälters
- 45: obere Seitenkanten des Transportbehälters
- 47: Drehachsfortsätze des Deckelements
- 49: Vorderkante des Deckelements
- 50: vordere Oberkante des geöffneten Transportbehälters
- 51: Griffelement des Deckelelements
- 53: Boden des Transportbehälters
- 55: Hilfsmittel
- 57: Aufnahmestruktur
- 59: Aufnahmen der Aufnahmestruktur
- 61: innere Bodenfläche des Transportbehälters
- 63: Verschlusselement
- 65: vorderes Ende des Verschlusselements
- 67: hinteres Ende des Verschlusselements
- 69: Ausnehmung des Deckelelements
- 71: magnetisches Öffnungselement des Sammelbehälters
- 73: Ausnehmung in der Nut
- 75: magnetisches Öffnungselement des Hilfsmittels
- 77: Anlageflächen des Hilfsmittels
- Bs: Breite des Sammelbehälters
- Ts: Tiefe des Sammelbehälters
- Hs: Höhe des Sammelbehälters
- B_{T}: Breite des Transportbehälters
- T_{T}: Tiefe des Transportbehälters
- H_{T}: Höhe des Transportbehälters
- S: Schwenkachse
- P: Schnittpunkt

## Patentansprüche

1. Transportsystem (1) für diagnostische Proben (3) mit einem Sammelbehälter (7) und mindestens einem mobilen Transportbehälter (5a, 5b), **dadurch gekennzeichnet, dass** der Transportbehälter (5a, 5b) eine verschließbare Oberseite (29) aufweist, wobei der Sammelbehälter (7) eine oberseitige Öffnung (11) aufweist, die mindestens so groß ist wie die Oberseite (29) des Transportbehälters (5a, 5b), wobei der Transportbehälter (5a, 5b) zum Ausleeren von diagnostischen Proben (3) in den Sammelbehälter (7) umgedreht mit verschlossener Oberseite (29) nach unten auf die oberseitige Öffnung (11) des Sammelbehälters (7) in eine eindeutig definierte Ausleerposition platzierbar ist, wobei die verschlossene Oberseite (29) des Transportbehälters (5a, 5b) nur in der Ausleerposition ohne ein Hilfsmittel (55) öffenbar ist und außerhalb der Ausleerposition nur mit dem Hilfsmittel (55) zerstörungsfrei öffenbar ist.

2. Transportsystem (1) nach Anspruch 1, wobei der Transportbehälter (5a, 5b) eine innere Bodenfläche (61) aufweist und die verschließbare Oberseite (29) mindestens so groß ist wie die innere Bodenfläche (61).

3. Transportsystem (1) nach Anspruch 1 oder 2, wobei der Transportbehälter (5a, 5b) eine Aufnahmestruktur (57) zur Aufnahme von diagnostischen Proben (3) aufweist, wobei die Aufnahmestruktur (57) eine Mehrzahl von Aufnahmen (59) aufweist, in denen eine Mehrzahl von diagnostischen Proben (3) im Wesentlichen parallel zueinander in einer im Wesentlichen vertikalen Ausrichtung anordbar sind.

4. Transportsystem (1) nach einem der vorhergehenden Ansprüche, wobei die verschließbare Oberseite (29) des Transportbehälters (5a, 5b) vollständig öffenbar ist.

5. Transportsystem (1) nach einem der vorhergehenden Ansprüche, der Transportbehälter (5a, 5b) ein vorzugsweise zumindest teilweise transparentes Deckelelement (31) aufweist, das unverlierbar und beweglich am Transportbehälter (5a, 5b) befestigt ist und in einer Schließposition die Oberseite (29) des Transportbehälters (5a, 5b) verschließt und in einer Öffnungsposition an einer Vorderwand (33) des Transportbehälters (5a, 5b) im Wesentlichen parallel zu dieser angeordnet ist.

6. Transportsystem (1) nach Anspruch 5, wobei das Deckelelement (31) aus der Öffnungsposition um eine im Wesentlichen parallel zu einer von der Oberseite (29) und der Vorderwand (33) gebildeten vorderen Oberkante (50) verlaufende Schwenkachse (S) in eine im Wesentlichen horizontale Ausrichtung schwenkbar ist, aus welcher das Deckelelement (31) im Wesentlichen horizontal in die Schließposition schiebbar ist.

7. Transportsystem (1) nach Anspruch 5 oder 6, wobei der Transportbehälter (5a, 5b) mindestens zwei innenseitige Nuten (43) aufweist, in denen das Deckelelement (31) geführt ist, wobei die Nuten (43) jeweils entlang einer Seitenkante (45) verlaufen, die von der Oberseite (29) und einer Seitenwandung (35, 37) des Transportbehälters (5a, 5b) gebildet sind.

8. Transportsystem (1) nach einem der Ansprüche 5 bis 7, wobei der Transportbehälter (5a, 5b) mindestens ein Verschlusselement (63) mit einem vorderen festen Ende (65) und einem elastisch verbiegbaren hinteren Ende (67) aufweist, wobei das hintere Ende (67) in einen Bewegungspfad des Deckelelements (31) von der Öffnungsposition in die Schließposition ragt, sodass das hintere Ende (67) des Verschlusselements (63) auf dem Weg in die Schließposition vom Deckelelement (31) aus dem Bewegungspfad geschoben wird und bei Erreichen der Schließposition in eine Ausnehmung (69) des Deckelelements (31) verschließend einschnappt.

9. Transportsystem (1) nach Anspruch 8, wobei der Sammelbehälter (7) mindestens ein magnetisches Öffnungselement (71) an seiner oberseitigen Öffnung (11) aufweist, das derart angeordnet ist, dass es das hintere Ende (67) des Verschlusselements (63) aus der Ausnehmung (69) des Deckelelements (31) zieht, wenn sich der Transportbehälter (5a, 5b) in der Ausleerposition befindet.

10. Transportsystem (1) nach einem der vorhergehenden Ansprüche, ferner mit einem Hilfsmittel (55), wobei das Hilfsmittel (55) ein zumindest teilweise zum Transportbehälter (5a, 5b) korrespondierend geformtes Öffnungsmittel mit einem magnetischen Öffnungselement (75) an einer definierten Position ist, wobei das Öffnungsmittel (75) derart definiert an der verschlossenen Oberseite (29) des Transportbehälters (5a, 5b) platzierbar ist, dass die Oberseite (29) öffenbar ist, wenn sich der Transportbehälter (5a, 5b) außerhalb der Ausleerposition befindet.

11. Transportsystem (1) nach einem der vorhergehenden Ansprüche, wobei die oberseitige Öffnung (11) des Sammelbehälters (7) an mindestens drei Seitenwandungen (17, 19, 21) des Sammelbehälters (7) angrenzt.

12. Verfahren zum Sammeln und Transportieren von diagnostischen Proben (3) mit den Schritten:
• Einsetzen einer Mehrzahl von diagnostischen Proben (3) in eine Aufnahmestruktur (57) in mindestens einem Transportbehälter (5a, 5b) durch eine verschließbare Oberseite (29) des Transportbehälters (5a, 5b), sodass die Mehrzahl von diagnostischen Proben (3) im Wesentlichen parallel zueinander in einer im Wesentlichen vertikalen Ausrichtung im Transportbehälter (5a, 5b) angeordnet sind,
• Verschließen der Oberseite (29) des Transportbehälters (5a, 5b) für den Transport des Transportbehälters (5a, 5b) mit einem Deckelelement (31), wobei die verschlossene Oberseite (29) des Transportbehälters (5a, 5b) nur in einer Ausleerposition ohne ein Hilfsmittel (55) öffenbar ist,
• Transportieren des Transportbehälters (5a, 5b) zu einem Sammelbehälter (7),
• Umdrehen des Transportbehälters (5a, 5b) zum Ausleeren der Mehrzahl von diagnostischen Proben (3) in den Sammelbehälter (7) mit verschlossener Oberseite (29) nach unten,
• Platzieren des Transportbehälters (5a, 5b) auf eine oberseitige Öffnung (11) des Sammelbehälters (7) in die eindeutig definierte Ausleerposition, und
• Öffnen der Oberseite (29) des Transportbehälters (5a, 5b) ohne das Hilfsmittel, sodass die Mehrzahl von diagnostischen Proben (3) in den Sammelbehälter (7) fällt.

## Claims

1. Transport system (1) for diagnostic samples (3) with a collecting container (7) and at least one mobile transport container (5a, 5b), **characterized in that** the transport container (5a, 5b) has a closable upper side (29), wherein the collecting container (7) has an opening (11) on the top side thereof which is at least as large as the upper side (29) of the transport container (5a, 5b), wherein the transport container (5a, 5b) can be placed in a positively defined emptying position on the top opening (11) with closed upper side (29) facing downwards in order to empty diagnostic samples (3) into the collecting container (7), wherein the closed upper side (29) of the transport container (5a, 5b) can only be opened without an auxiliary device (55) in the emptying position, and when not in the emptying position can only be opened non-destructively with the auxiliary device (55).

2. Transport system (1) according to Claim 1, wherein the transport container (5a, 5b) has an inner base surface (61), and the closable upper side (29) is at least as large as the inner base surface (61).

3. Transport system (1) according to Claim 1 or 2, wherein the transport container (5a, 5b) has a holding structure (57) for accommodating diagnostic samples (3), wherein the holding structure (57) has a plurality of receptacles (59) in which a plurality of diagnostic samples (3) can be arranged substantially parallel to each other in a substantially vertical orientation.

4. Transport system (1) according to any one of the preceding claims, wherein the closable upper side (29) of the transport container (5a, 5b) can be opened fully.

5. Transport system (1) according to any one of the preceding claims, the transport container (5a, 5b) has a preferably at least partially transparent cover element (31) which is attached non-detachably and movably to the transport container (5a, 5b) and in a closed position closes the upper side (29) of the transport container (5a, 5b), and in an open position is arranged on a front wall (33) of the transport container (5a, 5b), substantially parallel thereto.

6. Transport system (1) according to Claim 5, wherein the cover element (31) is pivotable from the open position about a pivot axis (S) running substantially parallel to a front upper edge (50) formed by the upper side (29) and the front wall (33) into a substantially horizontal orientation, from which the cover element (31) can be pushed substantially horizontally into the closed position.

7. Transport system (1) according to Claim 5 or 6, wherein the transport container (5a, 5b) has at least two grooves (43) on the inside, in which the cover element (31) is guided, wherein the grooves (43) each extend along side edges (45), which are formed by the upper side (29) and a side wall (35, 37) of the transport container (5a, 5b).

8. Transport system (1) according to one of Claims 5 to 7, wherein the transport container (5a, 5b) has at least one closure element (63) with a firm front end (65) and an elastically bendable rear end (67), wherein the rear end (67) protrudes in a path of movement of the cover element (31) from the open position into the closed position, so that the rear end (67) of the closure element (63) is displaced from the path of movement on the way to the closed position by the cover element (31), and when the closed position is reached, snaps into place in a recess (69) in the cover element (31).

9. Transport system (1) according to Claim 8, wherein the collecting container (7) has at least one magnetic opening element (71) at its top opening (11), which is arranged such that it pulls the rear end (67) of the closure element (63) out of the recess (69) in the cover element (31) when the transport container (5a, 5b) is in the emptying position.

10. Transport system (1) according to any one of the preceding claims, further comprising an auxiliary device (55), wherein the auxiliary device (55) is an opening means which is at least partially shaped to correspond to the transport container (5a, 5b) with a magnetic opening element (75) at a defined position, wherein the opening means (75) can be placed on the closed upper side (29) of the transport container (5a, 5b) in a defined manner such that the upper side (29) can be opened when the transport container (5a, 5b) is not in the emptying position.

11. Transport system (1) according to any one of the preceding claims, wherein the top opening (11) of the collecting container (7) adjoins at least three side walls (17, 19, 21) of the collecting container (7).

12. Method for collecting and transporting diagnostic samples (3) with the steps:
• Inserting a plurality of diagnostic samples (3) into a holding structure (57) in at least one transport container (5a, 5b) through a closable upper side (29) of the transport container (5a, 5b) such that the plurality of diagnostic samples (3) are arranged substantially parallel to each other in a substantially vertical orientation in the transport container (5a, 5b),
• Closing the upper side (29) of the transport container (5a, 5b) with a cover element (31) for transporting the transport container (5a, 5b), wherein the closed upper side (29) of the transport container (5a, 5b) can only be opened without an auxiliary device (55) when in an emptying position,
• Transporting the transport container (5a, 5b) to a collecting container (7),
• Turning the transport container (5a, 5b) over with the closed upper side (29) facing downwards to empty the plurality of diagnostic samples (3) into the collecting container (7),
• Placing the transport container (5a, 5b) in the positively defined emptying position on an opening (11) on the top of the collecting container (7), and
• Opening the upper side (29) of the transport container (5a, 5b) without the auxiliary device, so that the plurality of diagnostic samples (3) falls into the collecting container (7).

## Revendications

1. Système de transport (1) pour des échantillons de diagnostic (3) comprenant un récipient de collecte (7) et au moins un récipient de transport mobile (5a, 5b), **caractérisé en ce que** le récipient de transport (5a, 5b) présente un dessus pouvant être fermé (29), dans lequel le récipient de collecte (7) présente une ouverture de dessus (11) qui est au moins aussi grande que le dessus (29) du récipient de transport (5a, 5b), dans lequel le récipient de transport (5a, 5b), pour vider les échantillons de diagnostic (3) dans le récipient de collecte (7), peut être placé tourné avec le dessus (29) fermé orienté vers le bas sur l'ouverture de dessus (11) du récipient de collecte (7) dans une position de vidage définie clairement, dans lequel le dessus fermé (29) du récipient de transport mobile (5a, 5b) ne peut être ouvert sans un outil (55) que dans la position de vidage et ne peut être ouvert sans destruction qu'avec l'outil (55) en-dehors de la position de vidage.

2. Système de transport (1) selon la revendication 1, dans lequel le récipient de transport (5a, 5b) présente une surface de fond intérieure (61) et le dessus pouvant être fermé (29) est au moins aussi grand que la surface de fond intérieure (61).

3. Système de transport (1) selon la revendication 1 ou 2, dans lequel le récipient de transport (5a, 5b) présente une structure de réception (57) pour recevoir des échantillons de diagnostic (3), dans lequel la structure de réception (57) présente une pluralité de réceptions (59) dans lesquelles une pluralité d'échantillons de diagnostic (3) peuvent être agencés essentiellement parallèles entre eux dans une orientation essentiellement verticale.

4. Système de transport (1) selon l'une des revendications précédentes, dans lequel le dessus pouvant être fermé (29) du récipient de transport (5a, 5b) peut être ouvert complètement.

5. Système de transport (1) selon l'une des revendications précédentes, dans lequel le récipient de transport (5a, 5b) présente un élément de couvercle (31) essentiellement au moins partiellement transparent qui est fixé de manière imperdable et mobile au récipient de transport (5a, 5b) et qui, dans une position de fermeture, ferme le dessus (29) du récipient de transport (5a, 5b) et, dans une position d'ouverture, est disposé contre une paroi avant (33) du récipient de transport (5a, 5b), essentiellement parallèlement à celle-ci.

6. Système de transport (1) selon la revendication 5, dans lequel l'élément de couvercle (31) peut être pivoté de la position d'ouverture sur un pivot (S) essentiellement parallèle à une arête supérieure avant (50) formée par le dessus (29) et la paroi avant (33), dans une orientation essentiellement horizontale, à partir de laquelle l'élément de couvercle (31) peut être déplacé essentiellement horizontalement dans la position de fermeture.

7. Système de transport (1) selon la revendication 5 ou 6, dans lequel le récipient de transport (5a, 5b) présente au moins deux rainures côté intérieur (43) dans lesquelles l'élément de couvercle (31) est dirigé, dans lequel les rainures (43) passent respectivement le long d'une arête latérale (45) qui est formée par le dessus (29) et une paroi latérale (35, 37) du récipient de transport (5a, 5b).

8. Système de transport (1) selon l'une des revendications 5 à 7, dans lequel le récipient de transport (5a, 5b) présente au moins un élément de fermeture (63) avec une extrémité avant (65) fixe et une extrémité arrière (67) à flexibilité élastique, dans lequel l'extrémité arrière (67) fait saillie dans un trajet de mouvement de l'élément de couvercle (31) de la position d'ouverture à la position de fermeture de façon à ce que l'extrémité arrière (67) de l'élément de fermeture (63) soit poussée sur le trajet dans la position de fermeture de l'élément de couvercle (31) en-dehors du trajet de mouvement et que lorsque la position de fermeture est atteinte, se bloque et ferme par encliquetage dans un évidement (69) de l'élément de couvercle (31).

9. Système de transport (1) selon la revendication 8, dans lequel le récipient de collecte (7) présente au moins un élément d'ouverture magnétique (71) sur son ouverture de dessus (11) qui est ainsi disposé qu'il tire l'extrémité arrière (67) de l'élément de fermeture (63) hors de l'évidement (69) de l'élément de couvercle (31) lorsque le récipient de transport (5a, 5b) se trouve dans la position de vidage.

10. Système de transport (1) selon l'une des revendications précédentes, en outre avec un outil (55), dans lequel l'outil (55) est un moyen d'ouverture formé au moins partiellement de manière correspondante au récipient de transport (5a, 5b) comprenant un élément d'ouverture magnétique (75) à une position définie, dans lequel le moyen d'ouverture (75) peut être ainsi placé de manière définie sur le dessus (29) fermé du récipient de transport (5a, 5b) que le dessus (29) peut être ouvert lorsque le récipient de transport (5a, 5b) se trouve en-dehors de la position de vidage.

11. Système de transport (1) selon l'une des revendications précédentes, dans lequel l'ouverture de dessus (11) du récipient de collecte (7) est délimitée sur au moins trois parois latérales (17, 19, 21) du récipient de collecte (7).

12. Procédé de collecte et de transport d'échantillons de diagnostic (3) comprenant les étapes :
• d'insertion d'une pluralité d'échantillons de diagnostic (3) dans une structure de réception (57) dans au moins un récipient de transport (5a, 5b) à travers un dessus (29) pouvant être fermé du récipient de transport (5a, 5b) de façon à ce que la pluralité d'échantillons de diagnostic (3) soient disposés essentiellement parallèles entre eux dans une orientation essentiellement verticale dans le récipient de transport (5a, 5b),
• de fermeture du dessus (29) du récipient de transport (5a, 5b) pour le transport du récipient de transport (5a, 5b) avec un élément de couvercle (31), dans lequel le dessus fermé (29) du récipient de transport (5a, 5b) ne peut être ouvert sans un outil (55) que dans une position de vidage,
• de transport du récipient de transport (5a, 5b) vers un récipient de collecte (7),
• de rotation du récipient de transport (5a, 5b) pour vider la pluralité d'échantillons de diagnostic (3) dans le récipient de collecte (7) avec le dessus (29) fermé orienté vers le bas,
• de placement du récipient de transport (5a, 5b) sur une ouverture de dessus (11) du récipient de collecte (7) dans la position de vidage évidente, et
• d'ouverture du dessus (29) du récipient de transport (5a, 5b) sans l'outil de façon à ce que la pluralité d'échantillons de diagnostic (3) tombe dans le récipient de collecte (7).
